# EUROPEAN PATENT SPECIFICATION

(11) **EP 3 691 612 B1**
(45) Date of publication and mention of the grant of the patent: **26.11.2025**
(21) Application number: 18864459.5
(22) Date of filing: 05.10.2018
(51) Int. Cl.: A61K 31/365, A61K 9/20, A61K 9/16, A61K 31/337, A61K 9/14

(54) **HIGH-STRENGTH ORAL TAXANE COMPOSITIONS AND METHODS**
HOCHFESTE ORALE TAXANZUSAMMENSETZUNGEN UND VERFAHREN
COMPOSITIONS ORALES DE TAXANE DE GRANDE PUISSANCE ET PROCÉDÉS

(30) Priority: 06.10.2017 US 201762569258 P
(43) Date of publication of application: 12.08.2020
(60) Divisional application: 25210496.3
(73) Proprietor: Health Hope Pharma HK Limited, Hong Kong SAR (HK)
(72) Inventor: LAU, Johnson Yiu-Nam, Houston, California 77057 (US); YOON, Weng Li, Hong Kong (CN); LEE, Ming Tsun, Hong Kong (CN); LI, Jiahao, Hong Kong (CN); CHAN, Denise So Bik, Hong Kong (CN); LAU, Johnson Yiu-Nam, Houston, Texas 77057 (US)
(74) Representative: Cattaneo, Elisabetta
(86) International application number: PCT/US2018/054552
(87) International publication number: WO 2019/071092

(56) References cited:
- WO-A1-2015/152433
- WO-A1-2015/152433
- WO-A1-2017/133662
- CN-A- 103 083 240
- CN-A- 103 083 240
- US-A1- 2014 004 192
- PIAO, H. ET AL.: "A pre-formulation study of a polymeric solid dispersion of paclitaxel prepared using a quasi-emulsion solvent diffusion method to improve the oral bioavailability in rats", DRUG DEVELOPMENT AND INDUSTRIAL PHARMACY, vol. 42, no. 3, 10 July 2015 (2015-07-10), pages 353 - 363, XP055693917, DOI: 10.3109/03639045.2015.1054398
- MISHRA, D. K. ET AL.: "Amorphous solid dispersion technique for improved drug delivery: basics to clinical applications", DRUG DELIVERY AND TRANSLATIONAL RESEARCH, vol. 5, 2015, pages 552 - 565, XP035968796, DOI: 10.1007/s13346-015-0256-9

## Description

### Field of the Invention

The field of the invention is pharmaceutical compositions and scalable methods for manufacture of forms that provide oral delivery of taxanes at high bioavailability and high strength.

### Background

Taxanes are an important class of cytotoxic agents that includes paclitaxel (Taxol^{®}), docetaxel (Taxotere^{®} or Docecad), cabazitaxel, larotaxel, ortataxel, tesetaxel and the like. Paclitaxel is a diterpene isolated from the Pacific yew tree (Taxus brevifolia). Because paclitaxel binds tubulin, paclitaxel has the ability to inhibit cell division. Accordingly, paclitaxel has been approved for the treatment of ovarian, breast, lung, head and neck, and pancreatic cancers. Additionally, paclitaxel may effectively treat other maladies such as malaria and kidney disease. However, paclitaxel has a very low solubility in water, which makes formulating safe and effective therapies difficult.

In one known approach to improve solubility of taxanes for injectables, paclitaxel formulations include Cremophor^{®} EL (Kolliphor^{®} EL, polyoxyl 35 castor oil) or Tween^{®} 80 (polysorbate 80) and ethanol. When administered in such formulations Cremophor^{®} EL (or Tween^{®} 80) are independently toxic and exhibit side effects such as vasodilation, hypotension, labored breathing, lethargy, anaphylactoid hypersensitivity reactions, hyperlipidemia, abnormal lipoprotein patterns, aggregation of erythrocytes, and peripheral neuropathy. One option to avoid these side-effects is to administer the pharmaceutical composition orally. Unfortunately, when administered orally, such paclitaxel formulations suffer from very low bioavailability and absorption.

Docetaxel is a derivative of paclitaxel, which is approved for the treatment of breast, lung, prostate, stomach, and head and neck cancers. Typically, docetaxel is again formulated with surfactants for parenteral delivery, and like paclitaxel, the bioavailability of orally administered docetaxel is very low.

One reason for the low bioavailability may be that the taxanes are effluxed from target cells by multidrug transporters, such as P-glycoprotein (PGP). To address this problem, attempts have been made to co-administer taxanes with PGP inhibitors (e.g., HM30181A, ritonavir, and ketoconazole). See, for example, United State Patent No. 6,245,805 (to Broder et al.), United State Patent No. 7,041,640 (to Broder et al.), and United State Patent No. 7,115,565 (to Gao et al.).

Another reason for the low bioavailability is the poor solubility of taxanes in water. In an effort to enhance the solubility of taxanes, various techniques have been developed. For example, United States Patent Application Publication No. 2011/0,207,804 A1 (to Slotervaart Participaties BV) describes a solid dispersion formulation filled in capsules, and Chinese Patent Application No. CN103083240 A (to Shenyang Pharmaceutical University) describes using poor solvents and liquid bridging solvents in order to form an emulsion, which is subsequently used to form a dispersion of solid microspheres.

While many of these compositions can provide some improvement in supersaturation solubility and bioavailability of the taxanes, such formulations do not present a practical and convenient final dosage form for clinical dosing. Typically, solid powder dispersions are considered to be 'intermediate products' rather than 'finished products', being very light and fluffy in nature owing to its low bulk density. When filled into capsules, due to the limitations of the volume of powder that can be accommodated within the practical dimensions of an oral capsule, only a very limited loading of active per capsule can be achieved. For example, United States Patent Application Publication No. 2011/0,207,804 A1 (to Slotervaart Participaties BV) describes solid dispersion formulation in capsules providing only 15 mg of docetaxel in a capsule.

Alternatively the dosage form can be a tablet, given that the compacted nature of the excipients would allow a higher loading of the active ingredient per unit dosage form than a solid-dispersion powder in capsule. For example, it is conceivable that in a 1 g tablet that could afford 10% active ingredient loading, such that a 100 mg taxanes active loading can be attainable. For example, the approach adopted in Chinese Patent Application No. CN103083240 A requires the use of poor solvents and liquid bridging solvents in order to form an emulsion that is used to form a dispersion of solid microspheres. Unfortunately such microspheres are not suitable for compression into a high dosage form. The microspheres resulting from such a process have hollow centers, compression of which affects the integrity of their structure. This in turn negatively impacts the release of the active ingredient. As a result such microspheres are typically filled, without compression, into capsules. In addition the formulation disclosed in Chinese Patent Application No. CN103083240 A relies on the inclusion of a 'dispersant' (such as porous silica) to increase bioavailability of the active ingredient. In preferred embodiments up to 35% silica content is required. The requirement for the inclusion of a substantial amount of this excipient (or a similar excipient) necessarily limits the ability of such a formulation to achieve a high active loading content. It should also be appreciated that the preferred liquid bridging solvent is taught as methylene chloride. This organic solvent is classified as a Class 2 Solvent by the U.S Department of Health and Human Services, Food and Drug Administration (https://www.fda.gov/downloads/drugs/guidances/ucm073395.pdf) . According to the guidance, the use of Class 2 solvents should be limited in pharmaceutical product during to their inherent toxicity, having a Permissible Daily Exposure (PDE) of 6 mg/day. In addition, the good solvent/poor solvent method taught to produce the microspheres is relatively slow and non-scalable, and as such is not suitable for large scale manufacturing.

Tablet compositions of taxanes are described in International Patent Application Publication No. WO 2015/152433 A1 (to Hanmi Phar. Co. Ltd). Unfortunately only a 30 mg Paclitaxel strength in a total tablet weight of 750 mg was achieved. It should be appreciated that tablets intended for human use typically do not exceed 1 g, in order to ensure ease of swallowing. As the total dose of intravenously administered taxanes is often between 135-175 mg/m² (or about 300 mg for an individual having a body surface area (BSA) of 1.70 m²), and bioavailability of orally administered taxanes is lower than intravenously administered taxanes, it is foreseeable that the total oral dose required per patient will be > 300 mg in some instances. With known taxanes capsules and tablets having a strength of 15 to 30 mg, the number of unit doses that will need to be administered to a patient can easily exceed 10 capsules / tablets, which many patients cannot or will not ingest.

Considering potential clinical oral dosing regimens for taxanes, there is a strong, unmet need for improved tablet formulations of taxanes having higher dosage strengths and higher bioavailability. This is inherently technically challenging as higher concentrations of taxanes are thought to compromise solubility, and thus bioavailability.

### Summary of The Invention

The inventive subject matter provides high strength, high bioavailability, ready to administer oral compositions of taxanes that are manufacturable by readily scalable methods. The composition according to the invention is defined in claim 1. The method according to the invention is defined in claim 4. The ready to administer oral composition comprises a solid dispersion of docetaxel and a hypromellose acetate succinate (HPMCAS) carrier, which is compressed into a tablet form with high taxane loading. Docetaxel is present at a concentration of at least 6 wt%, more preferably at least 8 wt%, at least 10 wt%, or at least 13 wt%.

Docetaxel and the HPMCAS carrier are present in the oral composition at a ratio of between 1:0.5 and 1:4, inclusive.

It should be appreciated that the final dosage forms for oral taxanes described herein can advantageously and surprisingly offer high loading of active per dosage unit while maintaining high bioavailability, thereby enhancing patient acceptability of clinical oral dosing regimens for taxanes.

Additionally, it is contemplated that upon oral administration of oral compositions of the inventive subject matter, supersaturated solubility of at least 70% (or even at least 80% or at least 90%) of the taxanes released is maintained for a period of at least 2 hours (or at least 3 hours or even longer).

The oral compositions further include sodium dodecyl sulfate (SDS) at a ratio from 1:5 to 5:1 (e.g. 1:1).

The inventive subject matter also provides methods of producing a ready to administer oral composition of docetaxel, according to claim 4.

Various objects, features, aspects and advantages of the inventive subject matter will become more apparent from the following detailed description of preferred embodiments, along with the accompanying drawing figures in which like numerals represent like components.

### Brief Description of The Drawings

FIG. 1: Typical dissolution profiles of amorphous solid dispersions of Docetaxel:Povidone K-30:SDS (ASD Examples 1 to 3).
FIG. 2A: Typical dissolution profiles of amorphous solid dispersions of Docetaxel:HPMCP-50:SDS and HPMCP-55 (ASD Examples 4 and 5).
FIG. 2B: Typical dissolution profiles of amorphous solid dispersions of Docetaxel:HPMCP-50 and HPMCP-55 (without SDS, ASD Examples 6 and 7)).
FIG. 3A: Typical dissolution profiles of amorphous solid dispersions of Docetaxel:Eudragit:SDS (ASD Examples 8 to 10).
FIG. 3B: Typical dissolution profiles of amorphous solid dispersions of Docetaxel:Eudragit (without SDS, ASD Examples 11 and 12).
FIG. 4A: Typical dissolution profiles of amorphous solid dispersions of Docetaxel:HPMCAS:SDS (ASD Examples 13 to 17).
FIG. 4B: Typical dissolution profiles of amorphous solid dispersions of Docetaxel:HPMCAS (without SDS, ASD Examples 18 to 20).
FIG. 5: Typical dissolution profile of an amorphous solid dispersion (ASD) of Paclitaxel:HPMCAS:SDS (ASD Example 21).
FIG. 6A: Typical X-ray diffractograms of docetaxel amorphous solid dispersion (ASD Example 20) and crystalline docetaxel drug substance.
FIG. 6B: Typical X-ray diffractograms of a paclitaxel amorphous solid dispersion (ASD Example 21) and crystalline paclitaxel drug substance.
FIG. 7A: Typical dissolution profile of a docetaxel tablet prepared from an amorphous solid dispersion of the inventive concept: Docetaxel Tablet Example 1.
FIG. 7B: Typical dissolution profile of a docetaxel tablet prepared from an amorphous solid dispersion of the inventive concept: Docetaxel Tablet Example 2.
FIG. 7C: Typical dissolution profile of a docetaxel tablet prepared from an amorphous solid dispersion of the inventive concept: Docetaxel Tablet Example 3.
FIG. 7D: Typical dissolution profile of a docetaxel tablet prepared from an amorphous solid dispersion of the inventive concept: Docetaxel Tablet Example 4.
FIG. 7E: Typical dissolution profile of a docetaxel tablet prepared from an amorphous solid dispersion of the inventive concept: Docetaxel Tablet Example 5.
FIG. 7F: D Typical dissolution profile of a docetaxel tablet prepared from an amorphous solid dispersion: Docetaxel Tablet Example 6.
FIG. 8A: Typical dissolution profile of a paclitaxel tablet prepared from an amorphous solid dispersion: Paclitaxel Tablet Example 1.
FIG. 8B: Typical dissolution profile of a paclitaxel tablet prepared from an amorphous solid dispersion: Paclitaxel Tablet Example 2.
FIG. 8C: Typical dissolution profile of a paclitaxel tablet prepared from an amorphous solid dispersion: Paclitaxel Tablet Example 3.
FIG. 8D: Typical dissolution profile of a paclitaxel tablet prepared from an amorphous solid dispersion: Paclitaxel Tablet Example 4.
FIG. 8E: Typical dissolution profile of a paclitaxel tablet prepared from an amorphous solid dispersion: Paclitaxel Tablet Example 5.
FIG. 9: Comparative dissolution profiles of a paclitaxel prepared from an amorphous solid dispersion (Paclitaxel Tablet Example 5), a prior art paclitaxel tablet (Paclitaxel Tablet Comparator 1), and a prior art paclitaxel capsule (Paclitaxel Capsule Comparator 2).
FIG. 10: Typical dissolution profile of paclitaxel amorphous granules produced by fluid bed granulation.
FIG.11: Typical X-ray diffractograms of paclitaxel solid granule dispersion produced by fluid bed granulation and of a crystalline paclitaxel drug substance.
FIGs. 12A and 12B: FIG. 12A shows a typical dissolution profile of a 30 mg strength paclitaxel tablet produced from amorphous granules generated by fluid bed granulation. FIG. 12B shows a typical dissolution profile of a 90 mg strength paclitaxel tablet produced from amorphous granules generated by fluid bed granulation.

### Detailed Description

The inventive subject matter provides high-strength, ready to administer oral compositions of docetaxel, and methods of producing the same. The ready to administer oral compositions described herein comprise a compressed tablet with high taxane loading, which can be produced from an amorphous solid dispersion (ASD) of taxanes formed from a solution of taxanes and a hypromellose acetate succinate (HPMCAS) carrier. Methods of manufacture which are simple and amenable to commercial-scale production are also described. In chemotherapy, taxanes are generally administered as an intravenous drip over a period of one to several hours.

Surprisingly, Inventors have found that hypromellose acetate succinate (HPMCAS) can be used to provide a solid dispersion of a taxane that provides high dissolution in aqueous solutions and high bioavailability. Without wishing to be bound by theory Inventors believe that bioavailability is at least in part improved by pH-dependent dissolution of the HPMCAS carrier, which in turn provides at least partially selective release of active components of the composition (e.g. a taxane) in latter portions of the digestive tract (e.g. small and/or large intestine, where pH is higher than in the stomach) following ingestion. Different grades of HPMCAS dissolve at dissolution pHs ranging from about 5 to about 6 or about 6.8 or higher. As a result release of the majority or at least about half of the taxane active component of compositions of the inventive concept that have been placed in an aqueous solution occurs following a shift from highly acidic pH (i.e. below pH 5) to a pH of about 5 or higher (e.g. higher than about pH 5, higher than about pH 5.5, higher than about pH 6, and/or higher than about pH 6.5). The solid dispersion is manufactured as an amorphous solid, which can in turn be compressed into a tablet, caplet, or similar orally-administrable form that provides the taxane in a form and amount suitable for chemotherapy. In some embodiments the amorphous solid can be produced using spray-drying techniques. In other embodiments the amorphous solid can be produced by fluid bed granulation.

In some embodiments additional compounds can be incorporated into the solid dispersion. For example, one or more surfactants can be included in addition to the taxane and hypromellose acetate succinate. Suitable surfactants include anionic surfactants, cationic surfactants, and zwitterionic surfactants. In some embodiments the surfactant can be a hydrocarbon chain bearing a sulfate group or a salt thereof. The compositions according to the invention comprise at least sodium dodecyl sulfate.

Methods used in the production of amorphous solids, namely fluid bed granulation, generally incorporate the use of solvents during the manufacturing process. These solvents are removed or substantially removed in drying steps. While specific exemplary solvents are noted below, the any solvent suitable for pharmaceutical use can be used. Examples of such solvents include Class 2 and Class 3 solvents as described in FDA publication Q3C-Tables and List Guidance for Industry. This document is available at www.fda.gov/downloads/drugs/guidances/ucm073395.pdf (contents of which are incorporated herein by reference). In compositions of the inventive concept the concentration of a Class 2 solvent remaining following completion of processing should be an amount that provides less than the recommended PDE per day described therein using an effective dosing schedule. Similarly, in compositions of the inventive concept the amount of a Class 3 solvent remaining following completion of processing should be an amount that provides less than 50 mg per day using an effective dosing schedule.

### Formulation of High Strength - High Bioavailability Taxane Tablet Compositions

### Preparation of an Amorphous Taxane Dispersions by Spray-drying

### Spray Drying Methods

A spray solution, which includes one or more taxanes comprising docetaxel and a polymer carrier comprising HPMCAS was prepared by first dissolving the taxane(s) in a pharmaceutically acceptable organic solvent system. Suitable organic solvent systems include ethanol, acetone, tetrahydrofuran, and aqueous solutions of such organic solvents. The surfactant may be added to the spray solution.

The spray solution was supplied to a spray dryer (such as a Buchi Mini Spray Dryer B290^{™}), which was connected to a dehumidifier (such as a Buchi Dehumidifier), and an inert loop that enables the spray dryer to work with organic solvents safely (such as the Buchi Inert Loop B295^{™}) to generate an amorphous solid-dispersion powder.

The formation of the amorphous solid dispersion is affected by factors such as inlet temperature, outlet temperature, and atomization flow rate. In a typical operation an inlet temperature of from 80 °C to 120 °C and an outlet temperature of from 40 °C to 80 °C, can be used, provided that the outlet temperature is lower than the inlet temperature. In some embodiments the temperature difference between the inlet temperature and the outlet temperature can range from about 5 °C, about 7 °C, about 10 °C, about 15 °C , about 20 °C , about 25 °C , about 30 °C , about 35 °C , or about 40 °C. The atomization flow rate can range from about 10% to about 20% with 100% aspiration. For example, the spray solution can be provided to the spray dryer under the following conditions: an inlet temperature of 100 °C; an outlet temperature of 59 °C; and atomization flow rate of 15% with 100% aspiration.

The resulting spray dried amorphous solid dispersion are preferably stored under low moisture/humidity conditions. For examples, such a spray-dried amorphous solid dispersion can be stored in an air-tight container containing silica gel.

The surfactant was either spray-dried as part of the spray solution or, alternatively, physically mixed in with an amorphous solid dispersion prepared from a taxane and a polymer carrier as described above (for example, using a blender) prior to storage.

### Dissolution testing of spray-dried amorphous solid dispersion

The supersaturated solubility, physical stability, and release of taxanes from compositions were investigated using the USP 2011 dissolution apparatus II (Copley, UK) using dissolution medium equilibrated at 37 ± 0.5 °C in combination with simulated gastrointestinal fluids in the form of aqueous buffers having different pH values representative of different portions of the human gastrointestinal tract (e.g. stomach, small intestine, large intestine, and/or portions thereof). In order to simulate the changing pH conditions and transit times along the gastrointestinal tract, the following exemplary three-pH dissolution testing method at pH values of 4, 6.8 and 7.5 was used : (a) fill amorphous solid dispersion powder into a size 0 gelatin capsule(s); (b) set stirring speed of dissolution paddle to 100 rotations per minute (RPM); (c) introduce the capsule(s) (in a sinker device) to the dissolution vessel containing pH 4.0 dissolution medium for 1 hour, then adjust to pH 6.8 (by adding NaOH) for 1 hour, then adjust to pH 7.5 (by adding NaOH) for 2 hours; (d) collect samples at the following time points - 0, 10, 20, 30, and 60 minutes for pH 4; 70, 80, 90 and 120 minutes for pH 6.8; and : 130, 140, 150, 180, 210 and 240 minutes for pH 7.5, then replace an equivalent volume of fresh dissolution medium to compensate for loss due to each sampling; and (e) centrifuge the collected samples, and use the supernatants for HPLC analysis. In some embodiments such supernatants can be diluted in a suitable organic solvent prior to HPLC analysis (e.g. diluted 1:1 in methanol).

In an exemplary HPLC analysis of docetaxel preparations by dissolution testing (as described above), HPLC was performed using the following conditions:

| | |
|---|---|
| Column: | Stainless column (internal diameter 4.6 mm and length of 15 cm) packed with dimethyl-n-octadecylsilane stationary phase bonded to porous silica support for liquid chromatography (e.g. C18, 5 µm particle size) |
| Mobile phase: | A: 0.1% Formic acid in ultrapure water; |
| | B: 0.1% Formic acid in acetonitrile |
| Detector: | UV-absorption detector (absorbance at 230 nm) |
| Flow rate: | 1.2 mL/min |
| Injection volume: | 20 µL |
| Column temperature | 45 °C |
| Gradient system: | |

| **Time (min)** | **Eluent A (% by volume)** | **Eluent B (% by volume)** |
|---|---|---|
| 0.0 | 72.0 | 28.0 |
| 2.3 | 72.0 | 28.0 |
| 8.7 | 28.0 | 72.0 |
| 8.8 | 0.0 | 100.0 |
| 12.2 | 0.0 | 100.0 |
| 12.3 | 72.0 | 28.0 |
| 15.0 | 72.0 | 28.0 |

In an exemplary HPLC analysis of paclitaxel preparations by dissolution testing (as described above), HPLC was performed using the following conditions:

| | |
|---|---|
| Column: | Stainless column (internal diameter of about 4.6 mm and length of 15 cm) packed with dimethyl-n-octadecylsilane stationary phase bonded to porous silica support for liquid chromatography (e.g. C18, 5 µm particle size) |
| Mobile phase: | A: Ultrapure water; B: Acetonitrile |
| Detector: | UV-absorption detector (absorbance at 227 nm) |
| Flow rate: | 1.2 mL/min |
| Injection volume: | 20 µL |
| Column temperature: | 30 °C |
| Gradient system: | |

| **Time (min)** | **Eluent A (% by volume)** | **Eluent B (% by volume)** |
|---|---|---|
| 0.0 | 50.0 | 50.0 |
| 10.0 | 50.0 | 50.0 |
| 15.0 | 10.2 | 89.8 |
| 15.1 | 50.0 | 50.0 |
| 20.0 | 50.0 | 50.0 |

This "three pH dissolution model" spanning over 240 minutes is believed to be a more stringent screen to assess the effectiveness of amorphous compositions to prevent against the precipitation of taxanes in supersaturated concentrations as compared to single pH dissolution methods previously disclosed in PCT/KR2014/002734 (Hanmi Pharm. Co, Ltd) which is carried out over 120 minutes.

### Compositions of spray dried amorphous solid dispersion (ASD)

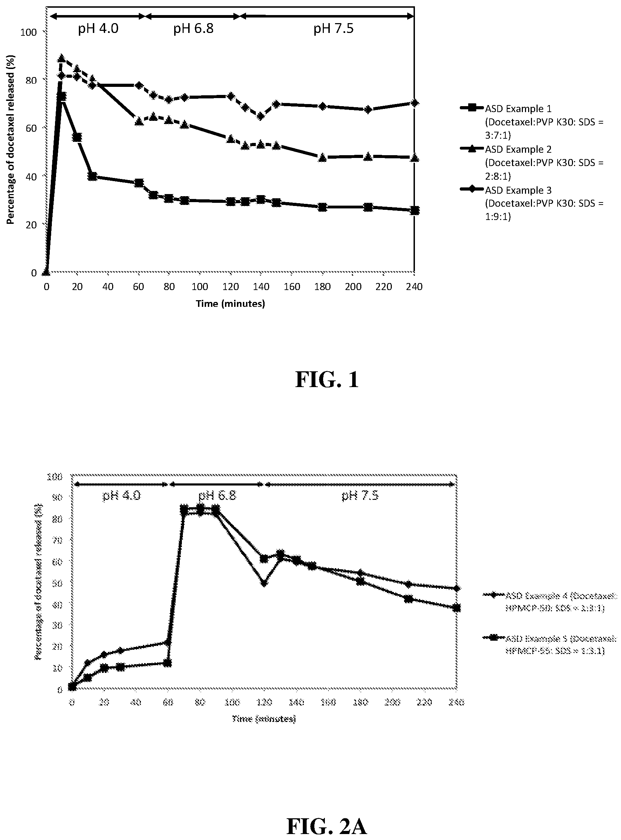

The compositions of the amorphous solid dispersion described above primarily comprise docetaxel as a taxane active component, HPMCAS as a polymer carrier, and SDS as a surfactant component. With the objective being to develop tablet compositions that provide high taxane active loading, it is necessary that the proportion of excipients be kept as low as possible when compared to the active ingredient. In the case of amorphous solid dispersions, the proportion of polymer carrier would also need to be minimized. This thinking, however, is contrary to conventional formulation principles, as higher polymer carrier to taxane ratios are conventionally thought to be necessary to maintain the supersaturated solubility of the active ingredient. This is evident from the data shown in Table 1, which summarizes the various compositions that utilize polyvinylpyrrolidone K-30 (PVP K-30), a polymer carrier which is used in known preparations of amorphous solid dispersion of taxanes (See e.g., US2011/0207804 A1 and PCT/KR2014/002734). FIG. 1, which shows the corresponding dissolution profiles (ASD Examples 1 to 3), clearly demonstrates that the content of PVP K-30 has to be as high relative to the active taxane component (for example as in a composition ratio of docetaxel: PVP K-30: SDS = 1:9:1 (ASD Example 3)) for supersaturated solubility to be sustainable in the dissolution model described. Due to the high proportion of PVP K-30 required, PVP K-30 is unsuitable to be used as a polymer carrier for an amorphous dispersion intended for a high-strength taxane tablet formulation.

**Table 1: Compositions of Docetaxel amorphous solid dispersions using PVP K-30 as polymeric carrier (with SDS) (not claimed)**

| | ASD Example 1 | ASD Example 2 | ASD Example 3 |
|---|---|---|---|
| | Docetaxel: PVP K-30: SDS ratio | | |
| | 3:7:1 | 2:8:1 | 1:9:1 |
| *Ingredients (g)* | | | |
| Docetaxel | 2.7 | 1.8 | 0.9 |
| PVP K-30 | 6.3 | 7.2 | 8.1 |
| SDS | 0.9 | 0.9 | 0.9 |

| *Volume of solvent (ml)* | | | |
|---|---|---|---|
| Ethanol | (200) | (200) | (200) |
| Water | (200) | (200) | (200) |
| Total | 9.9 | 9.9 | 9.9 |

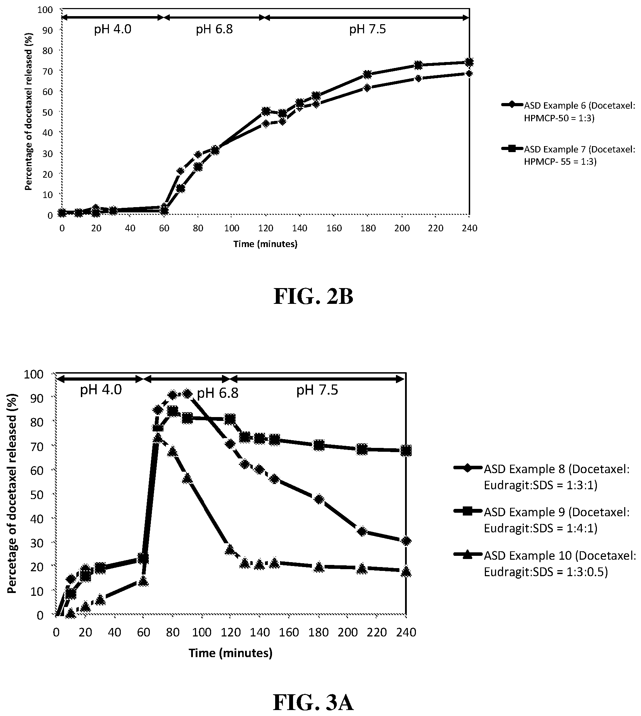

Tables 2A and 2B show compositions for ASD Examples 4 to 7, using Hydroxypropyl Methylcellulose Phthalate, HPMCP (50) and (55) respectively as alternative polymer carriers, with and without SDS as the surfactant component respectively. FIGs 2A and 2B show corresponding dissolutions profiles for these compositions. It should be appreciated that dissolution in the first, relatively acidic pH 4 step is minimal (substantially less than 50%) and increases rapidly on increasing to pH 6.8. This is found consistently with formulations of the inventive concept. FIG. 2A demonstrates that at a docetaxel: HPMCP: SDS ratio of 1:3:1, a sustained supersaturated solubility is not achieved. In the absence of SDS, release is also very slow, achieving maximum release after about 3 hours of dissolution of HPMCP polymers, as shown in FIG. 2B.

**Table 2A: Compositions of Docetaxel amorphous solid dispersions using HPMCP as polymeric carrier (with SDS) (not claimed)**

| | ASD Example 4 | ASD Example 5 |
|---|---|---|
| | Docetaxel: HPMCP: SDS ratio | |
| | 1:3:1 | 1:3:1 |
| *Ingredients (g)* | | |
| Docetaxel | 2 | 2 |
| HPMCP (50) | 6 | |
| HPMCP (55) | | 6 |

| *Volume of solvent (ml)* | | |
|---|---|---|
| Ethanol | (150) | (150) |
| Water | (37.5) | (37.5) |

| *Ingredients (g)* | | |
|---|---|---|
| SDS (physically mixed) | 2 | 2 |
| Total | 10 | 10 |

**Table 2B: Compositions of Docetaxel amorphous solid dispersions using HPMCP as polymeric carrier (without SDS) (not claimed)**

| | ASD Example 6 | ASD Example 7 |
|---|---|---|
| | Docetaxel: HPMCP ratio | |
| | 1:3 | 1:3 |
| *Ingredients (g)* | | |
| Docetaxel | 2.5 | 2.5 |
| HPMCP (50) | 7.5 | |
| HPMCP (55) | | 7.5 |

| *Volume of solvent (ml)* | | |
|---|---|---|
| Ethanol | (187.5) | (187.5) |
| Water | (46.9) | (46.9) |
| Total | 10 | 10 |

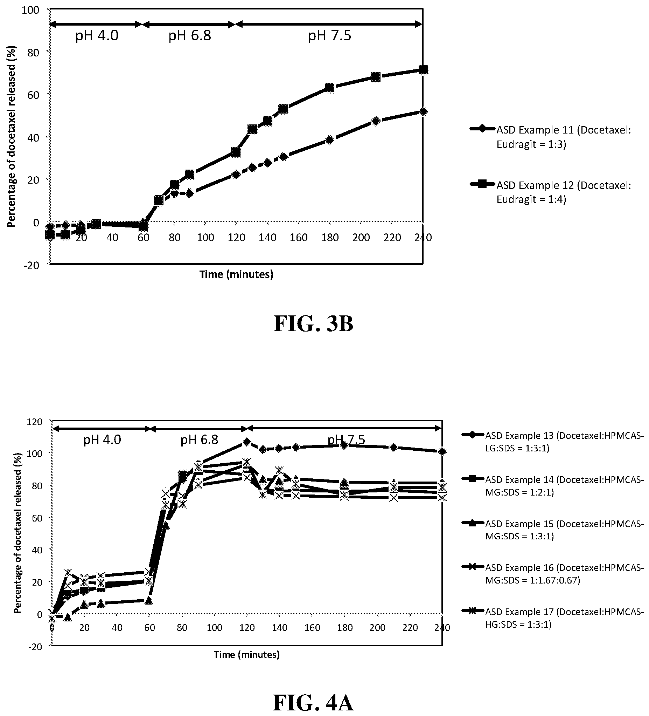

Tables 3A and 3B show the compositions using Eudragit L100-55, with and without SDS respectively, while FIGs 3A and 3B show the corresponding dissolution profiles. It is clear from FIG. 3A that a minimal docetaxel: Eudragit L100-55: SDS ratio that can successfully maintain supersaturated solubility is 1:4:1 (ASD Example 9). FIG. 3B shows that, while Eudragit L100-55 can be an effective polymer, when formulated without SDS the release of docetaxel was slow and started to achieve maximum concentration only after 180 minutes. Therefore, it appears that with compositions of taxanes including Eudragit, SDS can be a desirable component and can aid in the release of docetaxel in such formulations.

**Table 3A: Compositions of Docetaxel amorphous solid dispersions using Eudragit L100-55 as polymeric carrier (with SDS) (not claimed)**

| | ASD Example 8 | ASD Example 9 | ASD Example 10 |
|---|---|---|---|
| | Docetaxel: Eudragit L100-55: SDS ratio | | |
| | 1:3:1 | 1:4:1 | 1:3:0.5 |
| *Ingredients (g)* | | | |
| Docetaxel | 2 | 1.67 | 2.22 |
| Eudragit L100-55 | 6 | 6.67 | 6.66 |

| *Volume of solvent (ml)* | | | |
|---|---|---|---|
| Ethanol | (178.1) | (150) | (150) |
| Water | (9.4) | (37.5) | (37.5) |

| *Ingredients (g)* | | | |
|---|---|---|---|
| SDS (physically mixed) | 2 | 1.67 | 1.11 |
| Total | 10 | 10.01 | 9.99 |

**Table 3B: Compositions of amorphous solid dispersions using Eudragit.L100-55 as polymeric carrier (without SDS) (not claimed)**

| | ASD Example 11 | ASD Example 12 |
|---|---|---|
| | Docetaxel: Eudragit L100-55 ratio | |
| | 1:3 | 1:4 |
| *Ingredients (g)* | | |
| Docetaxel | 2.5 | 2 |
| Eudragit L100-55 | 7.5 | 8 |

| *Volume of solvent (ml)* | | |
|---|---|---|
| Ethanol | (222.6) | (179.9) |
| Water | (11.8) | (45) |
| Total | 10 | 10 |

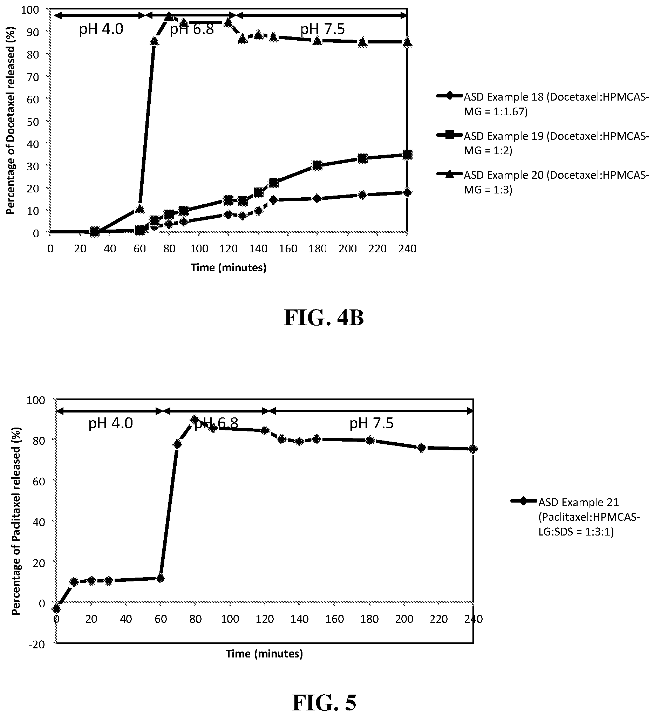

Tables 4A and 4B show the compositions using HPMCAS, with and without SDS respectively, while FIGs 4A and 4B show the corresponding dissolutions profiles. FIG. 4A, surprisingly demonstrated that with a very small proportion of HPMCAS (as in ASD Example 16, a docetaxel: HPMCAS: SDS ratio of 1: 1.67:0.67) can achieve a supersaturated state for docetaxel > 70% for more than 3 hours. Even more surprisingly, when HPMCAS and SDS proportions in the composition were increased slightly to docetaxel: HPMCAS-LG: SDS = 1:3:1 (ASD Example 13), the near supersaturated state of nearing to 100% of docetaxel released can be maintained for a sustained period.

**Table 4A: Compositions of Docetaxel amorphous solid dispersions using HPMCAS as polymeric carrier (with SDS)**

| | ASD Example 13 | ASD Example 14 | ASD Example 15 | ASD Example 16 | ASD Example 17 |
|---|---|---|---|---|---|
| | | | | | |
| | Docetaxel: HPMCAS: SDS ratio | | | | |
| | 1:3:1 | 1:2:1 | 1:3:1 | 1:1.67:0.67 | 1:3:1 |
| *Ingredients (g)* | | | | | |
| Docetaxel | 2 | 2.5 | 2 | 3 | 2 |
| HPMCAS-LG | 6 | - | - | - | |
| HPMCAS-MG | - | 5 | 6 | 5 | |
| HPMCAS-HG | - | - | - | - | 6 |

| *Volume of solvent (ml)* | | | | | |
|---|---|---|---|---|---|
| Ethanol | (150) | (150) | (131.3) | (150) | |
| Methanol | - | - | - | - | (200) |
| Water | (27.5) | (27.5) | (56.2) | (27.5) | |

| *Ingredients (g)* | | | | | |
|---|---|---|---|---|---|
| SDS (physically mixed) | 2 | 2.5 | 2 | 2 | 2 |
| Total | 10 | 10 | 10 | 10 | 10 |

**Table 4B: Compositions of Docetaxel amorphous solid dispersions using HPMCAS as polymeric carrier (without SDS) (not claimed)**

| | ASD Example 18 | ASD Example 19 | ASD Example 20 |
|---|---|---|---|
| | | | |
| | Docetaxel: HPMCAS ratio | | |
| | 1:1.67 | 1:2 | 1:3 |
| *Ingredients (q)* | | | |
| Docetaxel | 3.75 | 3.33 | 2.5 |
| HPMCAS-LG | | | |
| HPMCAS-MG | 6.26 | 6.66 | 7.5 |
| HPMCAS-HG | | | |

| *Volume of solvent (ml)* | | | |
|---|---|---|---|
| Ethanol | (187.7) | (199.8) | (164.1) |
| Water | (34.4) | (36.6) | (70.3) |
| Total | 10 | 10 | 10 |

A further unexpected observation from FIG. 4B was that in a composition with an HPMCAS content selected to give a docetaxel:HPCMAS ratio of 1:3 (ASD Example 20), SDS (i.e. the surfactant component) can be omitted from the composition of a high dose tablet taxane formulation while retaining its ability to maintain a sustained supersaturated concentration. As demonstrated with the formulation composition of docetaxel: HPMCAS of 1:3 and above, supersaturated solubility of 80% of docetaxel released can be maintained for at least 3 hours. This is in contrast with previous observations using other polymers described above (e.g. PVP K-30, Eudragit and HPMCP), where the surfactant has been necessary component. Surprisingly, with HPMCAS this is not the case. The lack of necessity for a surfactant component provides an additional advantage from the active ingredient loading point of view of a high loading tablet.

Table 5 and FIG. 5 show an example of the composition of an amorphous solid dispersion of Paclitaxel:HPMCAS:SDS (not claimed), as well as a typical corresponding dissolution profile. As observed with docetaxel, the amorphous solid dispersion based on Paclitaxel also yielded a sustained supersaturated state at a ratio of 1:3:1.

**Table 5: Compositions of Paclitaxel amorphous solid persions using HPMCAS as polymeric carrier (not claimed)**

| Ingredients (g) | ASD Example 21 |
|---|---|
| | |
| | Paclitaxel: HPMCAS: SDS ratio 1:3:1 |
| Paclitaxel | 2 |
| HPMCAS-LG | 6 |

| *Volume of solvent (ml)* | |
|---|---|
| Ethanol | (150) |
| Water | (27.5) |

| *Ingredients (q)* | |
|---|---|
| SDS (physically mixed) | 2 |
| Total | 10 |

### X-ray Diffraction Analysis

X-ray powder diffraction analysis of the docetaxel and paclitaxel amorphous solid dispersions prepared using a spray-dry method and respective drug substances were performed using the X'Pert PRO X-ray Diffraction System (Malvern Panalytical, United Kingdom). The samples were mounted on a sample holder and continuous scans over an angular range of 5° to 50° at 2θ were taken.

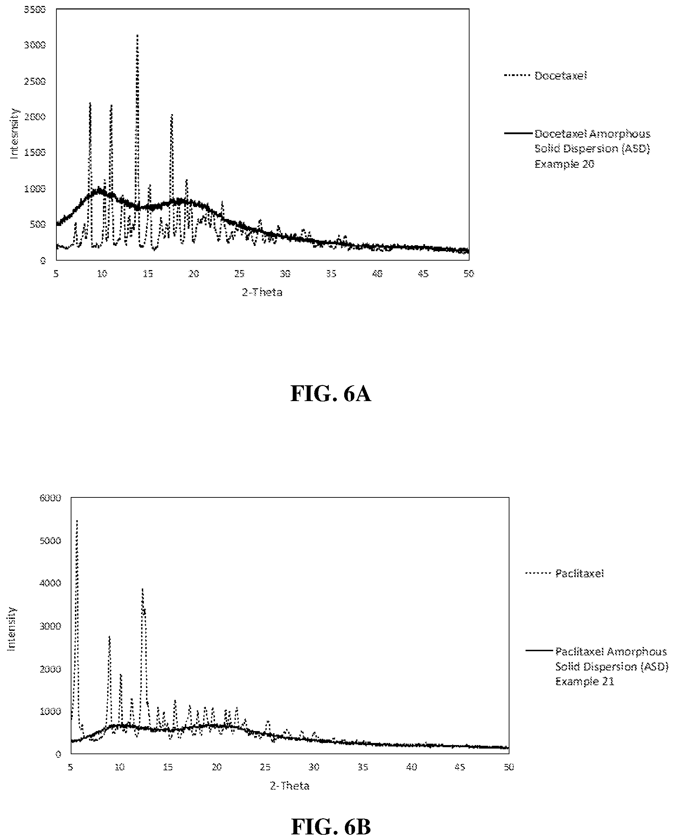

FIGs 6A and 6B, which show the X-ray diffractograms of amorphous solid dispersions of docetaxel and paclitaxel, show continuous X-ray diffraction patterns when compared to the X-ray diffractograms of the drug substance. This confirms that the conversion of the crystalline form of the drug substance to an amorphous form is provided in processes producing these amorphous solid dispersions.

### Method of Manufacture of tablets utilizing a spray-dried amorphous solid dispersion

A spray dried solid dispersion of the active ingredient was mixed with one or more intragranular excipients that can include a superdisintegrant and a binder, and combined with water using a wet granulation method to form a wet mass. Coarse screening was performed through a 1.68 mm (#12) sieve. The resulting granules were dried at 50°C followed by screening through a 0.84 mm (#20) sieve. The mixture was then combined with an extragranular portion of a superdisintegrant, a lubricant, and a filler in an extragranular fashion. The final mixture was then compressed into tablets using a tablet punch.

### Compositions of docetaxel tablets from spray-dried amorphous solid dispersion

Table 6 shows compositions of Docetaxel tablets produced from amorphous solid dispersion based on a Docetaxel: HPMCAS: Surfactant ratio of 1:2:1 and 1:3:1 with a range of active ingredient loadings and proportions of key functional excipients (such as superdisintegrants).

**Table 6 Example Compositions for High-Strength Docetaxel Tablet**

| | **Docetaxel Tablet Example 1** | **Docetaxel Tablet Example 2** | **Docetaxel Tablet Example 3** | **Docetaxel Tablet Example 4** | **Docetaxel Tablet Example** 5 | **Docetaxel Tablet Example 6** |
|---|---|---|---|---|---|---|
| | Percentage composition of ingredients (%) | | | | | |
| ***Amorphous Solid Dispersion*** | | | | | | |
| Docetaxel | 6.66 | 10.00 | 8.00 | 10.43 | 13.89 | 13.89 |
| HPMCAS-MG | 20.00 | 30.00 | 24.00 | 31.29 | 41.67 | 41.67 |
| SDS | 6.66 | 10.00 | 8.00 | 10.43 | 13.89 | 13.89 |
| Ethanol | (400) | (400) | (400) | (400) | (400) | (400) |
| Water | (100) | (100) | (100) | (100) | (100) | (100) |

| ***Intragranular Excipients*** | | | | | | |
|---|---|---|---|---|---|---|
| Crospovidone (PVPP) | - | - | - | 2.50 | 2.50 | 5.00 |
| Croscarmellose sodium | 2.50 | 2.50 | 2.50 | - | - | - |
| PVP-K30 | 5.00 | 5.00 | 5.00 | 5.00 | 5.00 | 5.00 |
| Water (wet granulation) | (5.5) | (5.5) | (5.5) | (5.0) | (6.0) | (8.9) |

| ***Extragranular Excipients*** | | | | | | |
|---|---|---|---|---|---|---|
| Crospovidone (PVPP) | - | - | - | 2.50 | 2.50 | 5.00 |
| Croscarmellose sodium | 2.50 | 2.50 | 2.50 | - | - | - |
| Microcrystalline cellulose | 54.98 | 38.80 | 48.50 | 36.71 | 19.93 | 15.10 |
| Sodium starch glycolate | 1.13 | 0.80 | 1.00 | 0.76 | 0.41 | 0.30 |
| Sodium stearyl fumarate | 0.57 | 0.40 | 0.50 | 0.38 | 0.21 | 0.15 |
| Total | 100.00 | 100.00 | 100.00 | 100.00 | 100.00 | 100.00 |

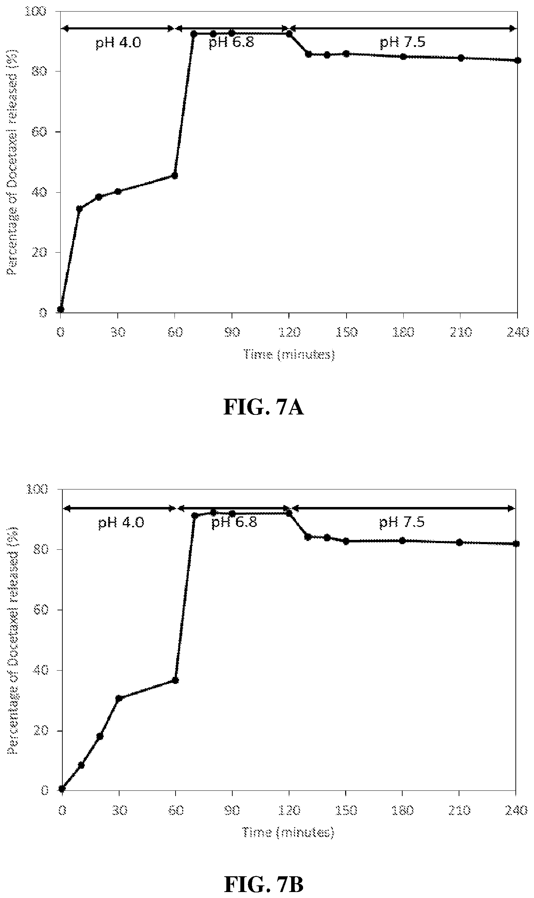

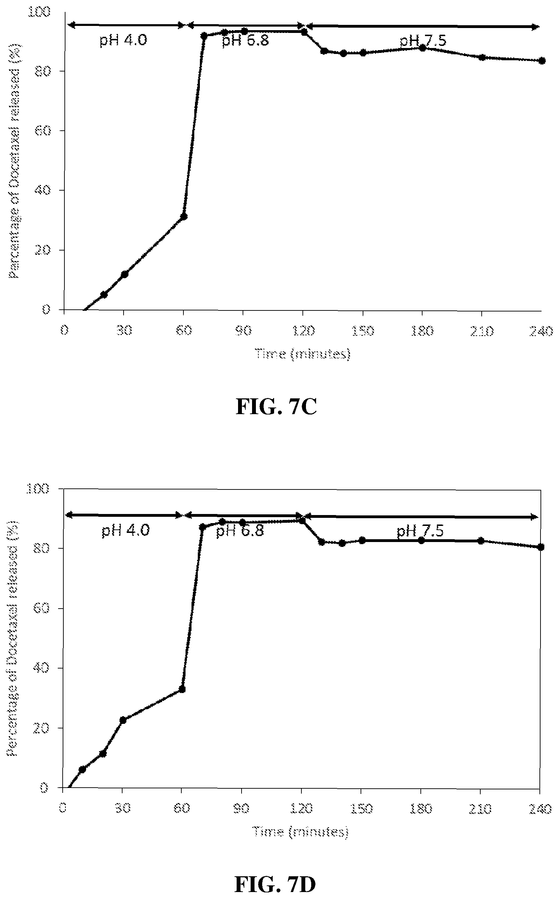

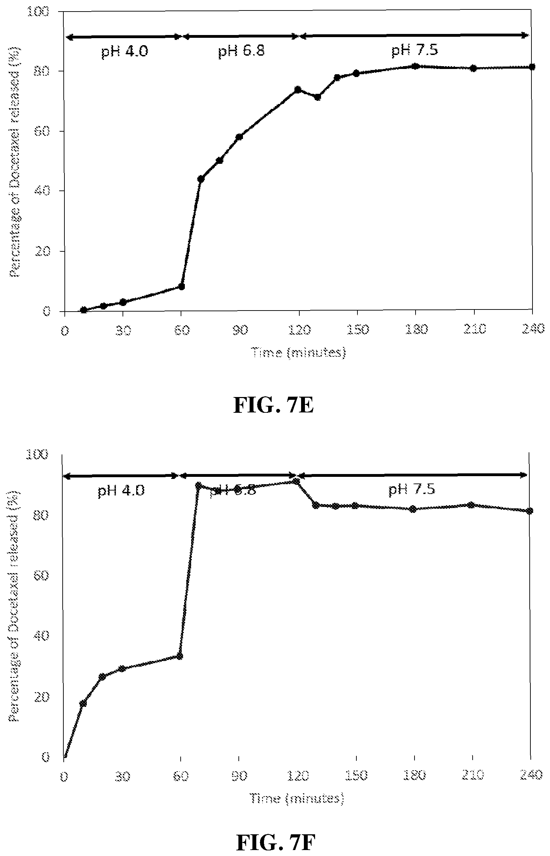

Based on solid dispersions of Docetaxel: HPMCAS: Surfactant at a ratio of 1:3:1, FIGs 7A to 7F show the dissolution curves of Docetaxel Tablet Examples 1 to 6, containing between 6.66% to 13.89% docetaxel. FIG. 7F shows that even when docetaxel loading was increased to 13.89% (Docetaxel Tablet Example 6), a formulation comprising HPMCAS was able to sustain a high supersaturated concentration of between 80 to 90% for at least 3 hours. It should be appreciated that formulations of the inventive concept were able to achieve 104.2 mg of active taxane in a 750 mg tablet (Docetaxel Tablet 6), for a loading of about 14% by weight of active component in the tablet. This represents an approximately 3.5-fold increase in loading relative to prior art formulations. The Inventor believes that higher loadings are possible with additional optimization. Active ingredient loadings of up to about 5%, 10%, 15%, 20%, and 25% and intermediate ranges between these values (e.g. 10% to 15%, 5% to 20%, etc.) are contemplated. It was noted that with increased concentration of HPMCAS (e.g. to about 40% of tablet content, as seen in Docetaxel Tablet Examples 5 and 6), the tablet has a tendency to form a gel layer which can retard the release of the active ingredient (see FIG. 7E) if the concentration of superdisintegrant is not adjusted accordingly. Therefore, for higher loadings of the active ingredient a concentration of superdisintegrant of at least 10% is preferable. Polyvinylpolypyrrolidone (PVPP), cross-linked modifications of polyvinylpyrrolidone, and croscarmellose sodium have also been identified as effective superdisintegrants to ensure effective release of active. It is contemplated that these disintegrants can be incorporated either intragranularly or extragranularly or both. It is also notable that while the preferred tablet compositions of taxanes described in International Patent Application Publication No. WO 2015/152433 A1 (to Hanmi Phar. Co. Ltd) could achieve 30 mg active in a tablets weighing 750 mg, our inventive tablets (as described in Docetaxel Example 5) can actually achieve 104.2 mg strength active in a same tablet weight of 750 mg. This marks an up to 3.47-fold increase in active loading per tablet compared to prior art examples.

From FIGS. 7A to 7F, it is apparent that in formulations incorporating HPMCAS-LG (which dissolves above pH 5.5), release of a significant proportion of active ingredient can occur after the shift of from pH 4 to 6.8. This advantageously reserves release of the majority of the taxane active ingredient for latter parts of the digestive process (e.g. after exit from the stomach). It should be appreciated that in some embodiments the formulation can be co-administered with a p-glycoprotein inhibitor (such as P-glycoproteins), so this pattern of release conferred by the formulation is actually desirable in order that absorption of taxanes can be maximized.

The Inventors contemplate that in some embodiments a tablet of the inventive concept can be enteric coated with enteric polymers which dissolves above pH 5.5, such as HPMCAS-LG, Hydroxypropyl Methylcellulose Phthalate, Eudragit L100-55, and/or Eudragit L100 .

### Compositions of paclitaxel tablets prepared from spray-dried amorphous solid dispersion

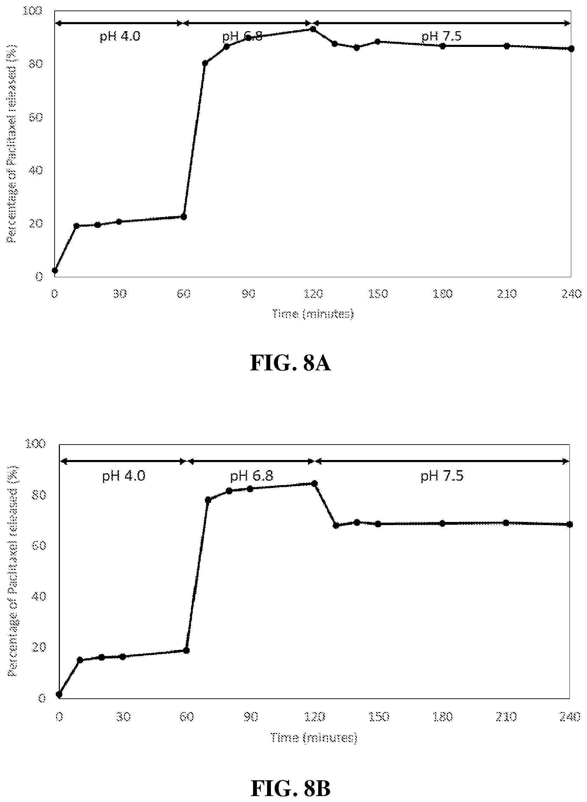

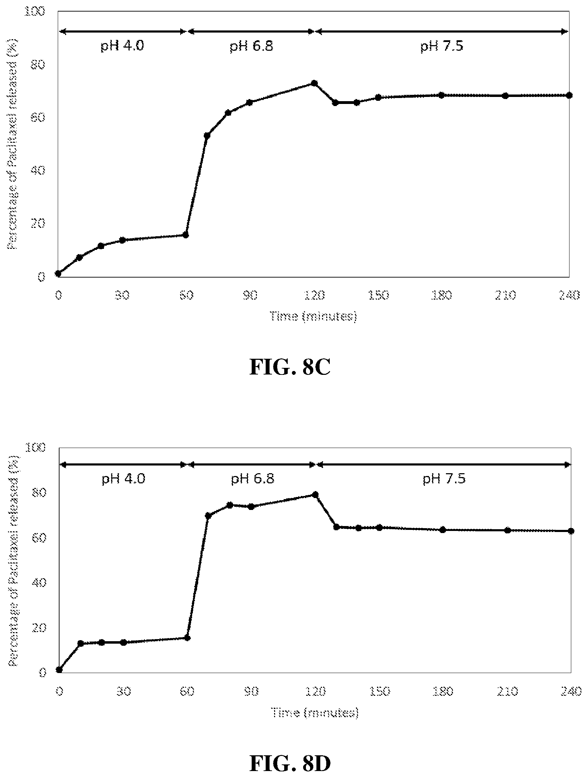

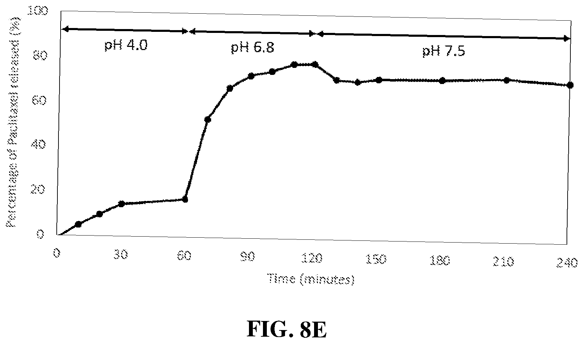

Table 7 shows the compositions of Paclitaxel tablets produced from amorphous solid dispersion based on Paclitaxel: HPMCAS:Surfactant with Paclitaxel loading from 6.66% to 11.11%. FIGs 8A to 8E show that supersaturation of Paclitaxel can also be maintained in these formulations for more than 3 hours, even when the tablet loading exceeds 10% (i.e. up to at least 11.11%). This is consistent with the data observed with docetaxel with polyvinylpolypyrrolidone and croscarmellose sodium being useful superdisintegrants, which can be incorporated either intragranularly or extragranularly or at both stages.

**Table 7: Example Compositions for High-Strength Paclitaxel Tablets (not claimed)**

| | **Paclitaxel** | **Paclitaxel** | **Paclitaxel** | **Paclitaxel** | **Paclitaxel** |
|---|---|---|---|---|---|
| | **Tablet Example 1** | **Tablet Example 2** | **Tablet Example 3** | **Tablet Example 4** | **Tablet Example 5** |
| | P ercentage co mposition of in igredients (%) | | | | |
| ***Amorphous Solid Dispersion*** | | | | | |
| Paclitaxel | 6.66 | 8.68 | 10.42 | 10.42 | 11.11 |
| HPMCAS-MG | 20.00 | 34.73 | 31.27 | 41.67 | - |
| HPMCAS-LG | - | - | - | - | 33.33 |
| SDS | 6.66 | 8.68 | 10.42 | 10.41 | 11.11 |
| Ethanol | (400) | (694.6) | (625.4) | (833.4) | (666.6) |
| Water | (100) | (173.65) | (156.35) | (208.35) | (166.65) |

| ***Intragranular Excipients*** | | | | | |
|---|---|---|---|---|---|
| Crospovidone (PVPP) | - | 5.00 | 4.00 | 5.00 | 5.00 |
| Croscarmellose sodium | 2.50 | 5.00 | - | - | - |
| PVP-K30 | 5.00 | 0.00 | 5.00 | 5.00 | 5.00 |
| Water (wet granulation) | (5.5) | (5) | (5) | (5) | (3.75) |

| ***Extragranular Excipients*** | | | | | |
|---|---|---|---|---|---|
| Crospovidone (PVPP) | - | 5.00 | - | 5.00 | 5.00 |
| Croscarmellose sodium | 2.50 | - | 4.00 | - | - |
| Microcrystalline cellulose | 54.98 | 31.98 | 33.84 | 21.84 | 28.58 |
| Sodium starch glycolate | 1.13 | 0.62 | 0.70 | 0.44 | 0.58 |
| Sodium stearyl fumarate | 0.57 | 0.31 | 0.35 | 0.22 | 0.29 |
| **Total** | 100.00 | 100.00 | 100.00 | 100.00 | 100.00 |

### Comparison Of Dissolution and Bioavailability Of Current Inventive Paclitaxel Tablet To Prior Art Compositions

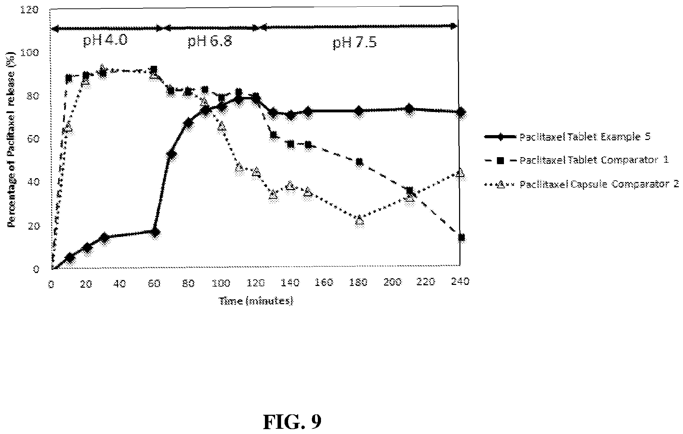

The dissolution profiles and oral bioavailabilities of Paclitaxel Tablet Example 5, a prior art paclitaxel tablet formulation (Paclitaxel Tablet Comparator 1), and a prior art paclitaxel capsule formulation (Paclitaxel Capsule Comparator 2) were compared. Typical dissolution profiles using the three pH method described above are shown in FIG. 9. Paclitaxel Tablet Comparator 1 has a loading of 30 mg active and includes the amorphous solid dispersion using PVP K-30 as polymer carrier, as described in Example 9 of International Patent Application No. WO 2015/152433 A1 (to Hanmi Phar. Co. Ltd), while Paclitaxel Capsule Comparator 2 is a 30 mg strength capsule in which Paclitaxel is solubilized in Polysorbate 80, as described in Comp. Ex 1 of International Patent Application No. WO 2015/152433 A1 (to Hanmi Phar. Co. Ltd).

For the dissolution study, 33.33 mg tablet strengths of Paclitaxel Example 5 was manufactured. Dissolution testing was then performed on Paclitaxel Tablet Example 5, Paclitaxel Tablet Comparator 1, and Paclitaxel Capsule Comparator 2. As shown in FIG. 9 the paclitaxel tablet formulation of the inventive concept exhibits delayed release at acidic pH (e.g. pH 4.0 or pH less than 6.8) and maintains high levels of saturation at pH 6.8 and above. FIG. 9 clearly demonstrates while the inventive tablet was able to maintain supersaturation of Paclitaxel for more then 3 hours, the supersaturated concentrations of Paclitaxel from the Paclitaxel Comparator Tablet 1 and Paclitaxel Capsule Comparator 2 started to decline after an hour, indicating that paclitaxel was starting to precipitate in these prior art compositions.

For the pharmacokinetic study, oral bioavailabilities of Paclitaxel Tablet Example 5, Paclitaxel Tablet Comparator 1, and Paclitaxel Capsule Comparator 2 were investigated by the co-administration of HM30181A tablet in naive Beagle dogs. Twenty naive male Beagle dogs (which weighed between 9.5 to 10.5 kg) were treated according to the regimen shown in Table 8. For the Paclitaxel Tablet Example 5, 80 mg strength tablets were manufactured while the Paclitaxel Tablet Comparator 1 and Paclitaxel Capsule Comparator 2 were available as 30 mg dose units and the targeted dose was between 80 to 90 mg per dog. Thirty minutes prior to the oral administration of each of these formulations, each dog was treated with a 15 mg HM30181A tablet. For the intravenous route, the dogs were infused with a 2 mL/kg single infusion of Paclitaxel solution (1 mg/ml, 4.9% Solutol HS15) over 60 minutes.

**Table 8 Study design of pharmacokinetic study**

| Group | Number of animals/gender | HM30181A (Dose and Route) | Paclitaxel (Dose and route) |
|---|---|---|---|
| 1 | 5 Male | 15 mg (1x15 mg), Oral | 80 mg (1 x 80 mg Paclitaxel Tablet Example 5), Oral |
| | | | |
| 2 | 5 Male | 15 mg (1x15 mg), Oral | 90 mg (3 x 30 mg Paclitaxel Tablet Comparator 1), Oral |
| 3 | 5 Male | 15 mg (1x15 mg), Oral | 90 mg (3 x 30 mg Paclitaxel Capsule Comparator 2) Oral |
| 4 | 5 Male | - | 2 mg/kg IV (infusion for 60 minutes) |

Table 9, which summarizes the pharmacokinetic data, clearly shows that the Paclitaxel Tablet Example 5 unexpectedly confers much improved oral absorption as evidenced by the increase in Cmax, AUC 0-t, AUC 0-∞ and F (%). Most notably, the bioavailability F(%) of this composition described for our invention was more than double that of both comparator formulations, despite affording a high loading of active per unit dosage form (i.e. use of less polymer carrier). This outcome, afforded by the tablet compositions described herein, is therefore unexpected.

**Table 9: Mean pharmacokinetic parameters of Paclitaxel following single intravenous or oral dose of Paclitaxel with HM30181A to male Beagle Dogs**

| Treatment | Tmax (hr) | Cₘₐₓ (ng/mL) | AUC₀₋ₜ (h·ng/mL) | AUC_{0-∞}(h·ng/mL) | MRT (hr) | t_{½} (hr) | V_{dss} (mL/kg) | CL (mL/hr/kg) | F (%) |
|---|---|---|---|---|---|---|---|---|---|
| HM30181A (1 x 15 mg tablet), oral followed by Paclitaxel Tablet Example 5, 80 mg (1 x 80 mg tablet), oral | 2.6 | 455 | 1777 | 1987 | 7.11 | 9.55 | - | - | 24.19 |
| HM30181A (1 x 15 mg tablet), oral followed by Paclitaxel Tablet Comparator 1, 90 mg (3 x 30 mg tablets), oral | 0.8 | 349 | 948 | 1018 | 3.20 | 4.70 | - | - | 11.47 |
| HM30181A (1 x 15 mg tablet), oral followed by Paclitaxel Capsule Comparator 2, 90 mg (3 x 30 mg capsules), oral | 0.6 | 442 | 903 | 1026 | 3.59 | 8.62 | - | - | 10.92 |
| Paclitaxel 2 mg/kg IV (Infusion for 60 minutes) | 1.0 | 1688 | 1836 | 1954 | 3.67 | 8.7 | 6587 | 1156 | - |

### Preparation and Characterization of Amorphous Taxane Compositions by Fluid Bed Granulation Fluid Bed Granulation Methods

Similar to the spray dry method, a spray solution containing a taxane can be prepared by first dissolving the taxane and HPMCAS in a suitable solvent system (e.g. aqueous ethanol). The surfactant ( sodium lauryl sulfate) can be added in the spray solution. Fluid bed granulation can be performed using any suitable fluid bed granulation system. Suitable fluid bed granulation systems include the Vector^{™} FLM-3 (Vector Corporation, Marion, USA) top-spray Fluidized Bed Granulator. In an exemplary embodiment a solution of taxane prepared as above was prepared and sprayed into a product container containing an intragranular excipient (such as microcrystalline cellulose) and one or more extra-granular excipient(s) (e.g. crosslinked polyvinyl pyrrolidone, colloidal silicon dioxide, sodium stearyl fumarate, microcrystalline cellulose, etc.) using the following parameters: spray rate ranging from 40 to 60 g/min, inlet temperature ranging from 60 °C to 70 °C, exhaust temperature ranging from 30 °C to 38 °C, and atomizing air pressure of 206.8 to 344.7 kPa (30 to 50 PSI). After the completion of the spraying process, the granulation was dried until a loss on drying (LOD) value of not more than 2.0% was achieved. When the taxane used was paclitaxel the resultant solid dispersion attained from such a process comprises free-flowing amorphous paclitaxel granules. Without wishing to be bound by theory, Inventors believe that the taxane active ingredient forms a coating or layer of amorphous matrix in HPMCAS over the intragranular excipient surface. Such amorphous granules can be formed into tablets providing a desired unit dose of taxane (e.g. from 1 mg to 100 mg or more). In such embodiments one or more surfactants can be provided in the formulation, for example by incorporation into the spray solution or addition as a solution or solid prior to drying. Similarly, in such embodiments one or more stabilizers can be incorporated by addition to the spray solution or addition as a solution or solid prior to drying. Such embodiments can also incorporate one or more disintegrants and/or superdisintegrants, for example by incorporation into the spray solution or by addition as a solution or solid prior to drying. Surfactants, stabilizers, and disintegrants/superdisintegrants suitable for use in spray drying are considered to be suitable for fluid bed granulation. **Table 10** shows the example compositions for 30 and 90 mg high-strength paclitaxel tablets produced using top-spray fluid bed granulation.

**Table 10 Exemplary compositions of High-Strength Paclitaxel Tablet (manufactured using top-spray fluid bed granulation) (not claimed)**

| | **Paclitaxel Tablet Example 6** | | |
|---|---|---|---|
| | **% w/w** | **mg of active /30 mg tablet** | **mg of active /90 mg tablet** |
| ***Spray solution*** | | | |
| Paclitaxel | 9.5 | 30.0 | 90.0 |
| HPMCAS-LG | 28.6 | 90.0 | 270.0 |
| Sodium lauryl sulfate | 9.5 | 30.0 | 90.0 |
| Ethanol 200 proof* | (364.2) | (1147.5) | (3442.5) |
| Sterile water* | (64.3) | (202.5) | (607.5) |

| ***Intragranular Excipients*** | | | |
|---|---|---|---|
| Microcrystalline cellulose | 35.5 | 111.9 | 335.7 |

| ***Extragranular Excipients*** | | | |
|---|---|---|---|
| Crospovidone | 9.5 | 30.0 | 90.0 |
| Colloidal silicon dioxide | 0.7 | 2.1 | 6.3 |
| Sodium stearyl fumarate | 1.9 | 6.0 | 18.0 |
| Microcrystalline cellulose | 4.8 | 15.0 | 45.0 |
| **Total** | **100.0** | **315.0** | **945.0** |

| | | | |
|---|---|---|---|
| *Not present in the finished product (removed during fluid bed granulation process) | | | |

### Dissolution Testing of Taxane Preparation Produced by Fluid Bed Granulation

Dissolution testing for amorphous solid dispersion and tablet compositions manufactured using a spray-dry method was performed using a three-pH model as described above. A similar four-pH dissolution model was utilized for characterizing dissolution of amorphous taxane-containing granules and tablets produced from amorphous taxane-containing granules produced by fluid bed granulation, by adding a further step of dissolution at pH 1.2. This provides a rigorous evaluation on the physical testing and release of taxanes from the composition and more closely simulates the changing pH of the GI tract that would be experienced by such compositions following ingestion. It should be appreciated that dissolution in the first acidic pH 1.2 and second acidic pH 4.0 steps is minimal (substantially less than 50%) and increases rapidly on increasing to pH 6.8. This is found consistently with formulations of the inventive concept.

An exemplary four-pH dissolution testing method utilizing simulated gastrointestinal fluids (in the form of aqueous buffers having different pH values representative of different portions of the human gastrointestinal tract- e.g. stomach, small intestine, large intestine, and/or portions thereof) at pH values of 1.2, 4.0, 6.8 and 7.5 can be performed as follows: (a) fill amorphous solid granule dispersion into a size 0 gelatin capsule/s or, alternatively, form a tablet containing amorphous taxane-containing granule dispersion; (b) set stirring speed of dissolution paddle to 100 rotations per minute (RPM); (c) introduce the capsule/s or tablet (in a sinker device) to a dissolution vessel containing 720 ml 0.1N hydrochloric acid (pH 1.2); (d) after 1 hour at pH 1.2, add 180 ml 0.25 M sodium phosphate monobasic (NaH₂PO₄) solution containing 5% w/v polysorbate 80 to the final molarity of 0.05 M NaH₂PO₄ and concentration of 1% w/v polysorbate 80 respectively, and adjust to pH 4.0 (by adding 20% sodium hydroxide (NaOH, 1% w/v polysorbate 80 was used to maintain submersion of the sinker device during dissolution); (e) after 1 hour at pH 4.0, adjust pH of the solution to pH 6.8 (e.g. by adding 20% NaOH); (f) after 1 hour at pH 6.8, adjust pH of the solution to pH 7.5 (e.g. by adding 20% NaOH) and stir for an additional 2 hours. Samples are collected at the following time points - 0, 10, 20, 30, and 60 minutes for pH 1.2; 70, 80, 90 and 120 minutes for pH 4; 130, 140, 150 and 180 minutes for pH 6.8; 190, 200, 210, 240, 270 and 300 minutes for pH 7.5. An equivalent volume of fresh dissolution medium is added after sampling to compensate for volume loss. The collected samples are centrifuged and the supernatants subjected to HPLC analysis. Such supernatants can be diluted in a suitable organic solvent prior to HPLC analysis (e.g. diluted 1:1 in methanol).

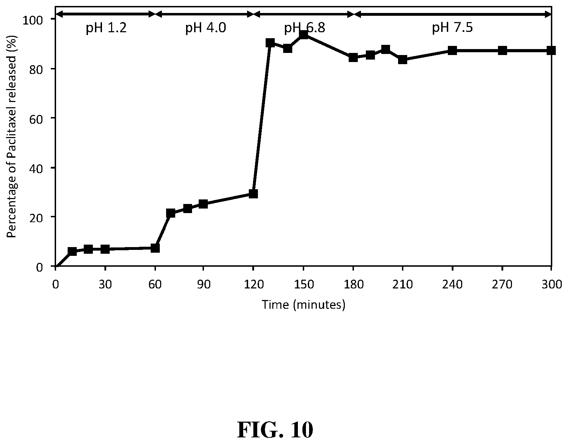

To characterize the dissolution of solid amorphous granule dispersion of paclitaxel equivalent to a high-strength tablet containing 90 mg of paclitaxel, 786 mg of amorphous paclitaxel granules were used and filled into four size 0 capsules and analyzed according to the procedure above. FIG. 10 shows a typical dissolution profile for such a solid amorphous granule dispersion. It is evident that the granules are able to sustain a supersaturated concentration of > 70% of active component (i.e. paclitaxel) released for at least 3 hours. From the improved solubility and supersaturated concentrations achieved, it can be inferred that paclitaxel has been converted into an amorphous state using the fluid bed granulation process.

### X-Ray Diffraction Analysis of Amorphous Granules Produced by Fluid Bed Granulation

X-ray powder diffraction analysis of the solid amorphous granule paclitaxel dispersions and conventional paclitaxel drug substance were performed using the X'Pert PRO^{™} X-ray Diffraction System (Malvern Panalytical, United Kingdom). The samples were mounted on a sample holder and continuous scans over an angular range of 5° to 50° at 2θ were taken.

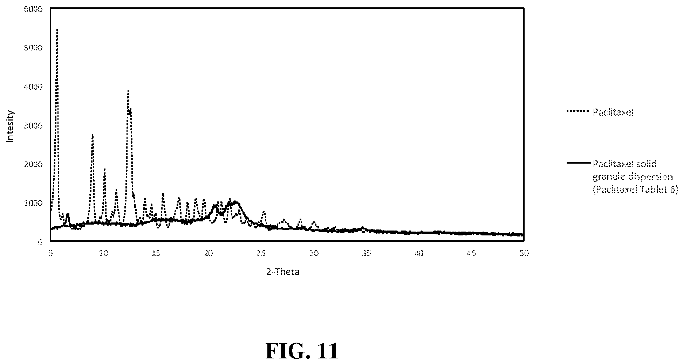

FIG. 11 shows a typical X-ray diffractogram of a solid granule amorphous dispersion of paclitaxel. The continuous appearance of the X-ray powder diffraction pattern when compared to conventional crystalline paclitaxel further confirms the conversion of the crystalline form of the drug substance to the amorphous form in the solid granule dispersions.

### Preparation of paclitaxel tablets from amorphous granules produced by fluid bed granulation and their dissolution profiles

In a typical process for preparing tablets from solid amorphous granules produced by fluid bed granulation as described above, the granules are milled and blended with one or more excipients. Typical excipients include disintegrants, fillers, binders, etc. examples of which are provided in Table 10. Tablets can be produced by any suitable process, for example molding and/or compression. Tablets can be produced in any suitable shape that facilitates ingestion, for example, round, ovoid, cylindrical, etc. Similarly, tablets can be produced at any suitable hardness or level of compression as needed to provide suitable cohesion and size. In the examples provided the 30 mg tablets were compressed to an average hardness of 8 kp while the 90 mg tablets to an average hardness of 12 kP.

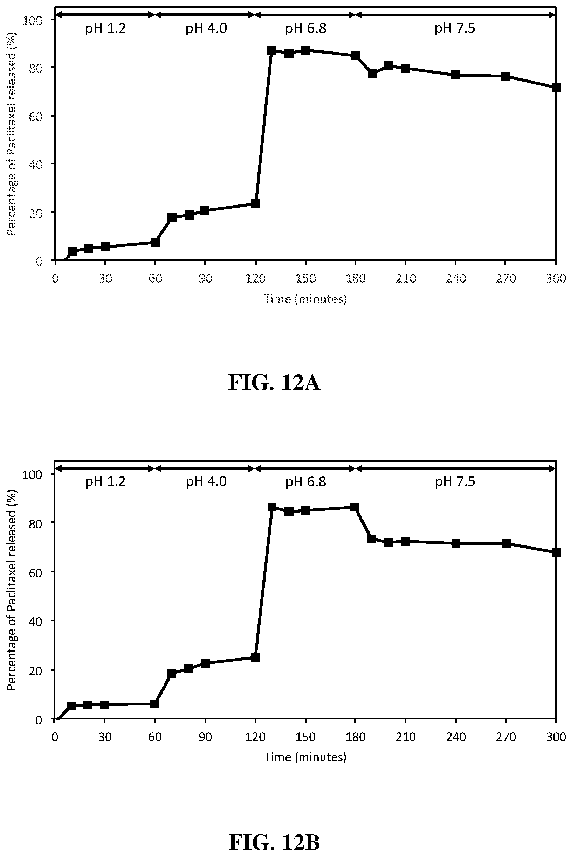

The dissolution profiles (determined using the four pH step process as described above) of 30 and 90 mg tablets are shown in FIGs. 12A and 12B, respectively. These dissolution profiles indicate that both tablets disintegrated and attained maximum release of the active within 10 minutes of the HPMCAS polymer dissolving after pH exceeds 4.0, and again providing a percentage release > 70% that was sustainable for at least 3 hours.

### Comparison of bioavailability of paclitaxel tablet produced from amorphous granules generated using fluid bed granulation to a prior art composition

The oral bioavailabilities of paclitaxel tablets prepared from amorphous granules generated by fluid bed granulation using HPMCAS polymer (specifically, top-spray fluid bed granulation) and prior art paclitaxel tablets were investigated with the co-administration of HM30181A tablet in naive beagle dogs. The prior art paclitaxel tablet has a loading of 30 mg of active ingredient and comprises an amorphous solid dispersion produced using PVP K-30 as a polymer carrier, as described in Example 9 of International Patent Application No. WO 2015/152433 A1 (to Hanmi Phar. Co. Ltd).

For the pharmacokinetic study, fifteen naive male Beagle dogs (which weighed between 9.0 to 10.5 kg) were randomly divided into 3 groups of 5 animals according to the study design shown in Table 11. The animals in Group 1 and 2 were treated with paclitaxel tablets prepared from amorphous granules generated by fluid bed granulation using HPMCAS polymer or prior art paclitaxel tablets, respectively, with co-administered with HM30181A for both tablet formulations in a cross-over design with two-phases separated by a one-week washout period. The animals in Group 3 were dosed with Paclitaxel intravenously in a single phase only.

**Table 11 Study design of pharmacokinetic study**

| Study design | Group | Number of animals/ gender | Treatment | | |
|---|---|---|---|---|---|
| | | | Phase 1 | Washout period | Phase 2 |
| Cross-over (Two-phase) | 1 | 5 male | HM30181A Tablet (1 x15 mg), Oral followed by Paclitaxel Tablet Example 6, 60 mg (2 x 30 mg), Oral | 1 week | HM30181A Tablet (1 x15 mg), Oral followed by Paclitaxel Comparator Tablet 1, 60 mg (2 x 30 mg), Oral |
| Cross-over (Two-phase) | 2 | 5 male | HM30181A Tablet (1 x15 mg), Oral followed by Paclitaxel Comparator Tablet 1, 60 mg (2 x 30 mg), Oral | | HM30181A Tablet (1 x15 mg), Oral followed by Paclitaxel Tablet Example 6, 60 mg (2 x 30 mg), Oral |
| Single-phase | 3 | 5 male | Paclitaxel, 2 mg/kg IV (infusion for 60 minutes) | - | - |

The oral dose selected for this study was 60 mg per dog, which is lower than the 80 to 90 mg dose used in the earlier pharmacokinetic study comparing a tablet manufactured by a spray-dry process. Therefore, two units of 30 mg strength tablets for the paclitaxel tablets prepared from amorphous granules generated by fluid bed granulation using HPMCAS polymer or the prior art paclitaxel tablets were used when dosing each dog in the appropriate testing groups. Thirty minutes prior to the oral administration of each of these formulations each dog was treated with a 15 mg HM30181A tablet. For the intravenous route, the dogs were infused with a 2 mL/kg single infusion of paclitaxel solution (1 mg/ml, 4.9% Solutol HS15^{™}) over 60 minutes.

Table 12 summarizes the pharmacokinetic data obtained, and shows that at a dose of 60 mg per dog, paclitaxel tablets prepared from amorphous granules generated by fluid bed granulation using HPMCAS polymer also confer improved oral absorption as evidenced by the increase in the AUC ₀₋ₜ, AUC_{0-∞} and F(%).

**Table 12: Mean pharmacokinetic parameters of Paclitaxel following cross-over dosing of Paclitaxel tablet Example 6 and Paclitaxel tablet Comparator 1 (with HM30181A) or single intravenous dose of Paclitaxel to male beagle dogs.**

| Treatment | Tmax (hr) | Cₘₐₓ (ng/mL) | AUC₀₋ₜ (h·ng/mL) | AUC_{0-∞} (h·ng/mL) | MRT₀₋ₜ (hr) | t_{½} (hr) | V_{dss} (mL/k g) | CL (mL/hr/kg) | F (%) |
|---|---|---|---|---|---|---|---|---|---|
| HM30181A (1 x 15 mg tablet), oral followed by Paclitaxel Tablet Example 6, 60 mg (2 x 30 mg tablets), oral | 1.25 | 114.53 | 488.73 | 596.40 | 7.54 | 8.97 | - | - | 23.27 |
| HM30181A (1 x 15 mg tablet), oral followed by Paclitaxel Tablet Comparator 1, 60 mg (2 x 30 mg tablets), oral | 0.90 | 166.79 | 458.53 | 517.87 | 6.69 | 8.67 | - | - | 21.00 |
| Paclitaxel 2 mg/kg IV (Infusion for 60 minutes) | 0.00 | 810.60 | 701.44 | 767.73 | 5.26 | 757 | 2103. 47 | 2658.00 | - |

Thus, various oral compositions of taxanes having high loading of active and high bioavailability have been provided herein. The described methods advantageously use spray drying processes or fluid bed granulation methods that do not involve the formation of an emulsion, poor solvents, or liquid bridging solvents, as required by prior art processes. Rather, in the processes described herein, the dissolved taxane are spray dried, resulting in the formation solid powder/ granule amorphous solid dispersions. Such solid dispersions, unlike hollow microspheres, can effectively be compressed into tablets as discussed herein.

It should be appreciated that compositions of the inventive can be effectively compressed into a tablet, a property that is not shared by compositions that incorporate the active ingredient into hollow core microspheres. Similarly it should be appreciated that compositions of the inventive concept are produced by scalable means that are well suited to large scale manufacturing, and do not require the inclusion of a dispersant (such as porous silica) to increase and/or provide adequate bioavailability. This provides a beneficial technical effect, as exclusion of such a dispersant permits higher loading of an active ingredient (such as a taxane) in the final dosage form.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

The inventive subject matter, therefore, is not to be restricted except in the scope of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

## Claims

1. A ready to administer oral composition, comprising: an amorphous solid dispersion comprising docetaxel, a hypromellose acetate succinate (HPMCAS) carrier; and sodium dodecyl sulfate (SDS), wherein the docetaxel, and the HPMCAS are present in the oral composition at a ratio of from 1:0.5 to 1:4 inclusive; wherein the docetaxel and the SDS are present in the oral composition at a ratio from 1:5 to 5:1, wherein the solid dispersion is a spray-dried amorphous solid dispersion or an amorphous granule produced by fluid bed granulation and wherein the docetaxel is present at a concentration of at least 6 wt% of the oral composition.

2. The composition of claim 1 , wherein the solid dispersion is mixed extragranularly with intragranular excipients via a wet granulation method to form a mixture,
wherein the mixture is selected from the group consisting of lubricant, a filler and a superdisintegrant to form a tablet mixture, and wherein the tablet mixture is compressed into tablets to form the ready to administer oral composition.

3. The composition of one of claim 1 or claim 2, further comprising a disintegrant or a superdisintegrant.

4. A method of producing a ready to administer oral composition, comprising:
formulating a solution comprising docetaxel, an HPMCAS carrier and SDS wherein docetaxel, and the HPMCAS are at a ratio of from 1:0.5 to 1:4, inclusive; and wherein the docetaxel and the SDS are at a ratio from 1:5 to 5:1, inclusive;
applying the solution to an intragranular excipient using a fluid bed granulation system to form amorphous granules comprising docetaxel and the HPMCAS carrier:
drying the plurality of amorphous granules; and
compressing the plurality of amorphous granules to form a tablet having docetaxel concentration of at least 6% by weight.

5. The method of claim 4, wherein the intragranular excipient is microcrystalline cellulose.

6. The method of claim 4 or claim 5, further comprising a step of adding an extragranular excipient prior to drying
wherein the extragranular excipient is selected from the group consisting of crosslinked polyvinylpyrrolidone, amorphous silicon dioxide, sodium stearyl fumarate, and microcrystalline cellulose.

7. The method of one of claims 4 to 6, wherein the solution further comprises a surfactant.

8. The method of one of claims 4 to 7, comprising the step of adding a stabilizer prior to compressing, wherein the stabilizer is selected from the group consisting of citric acid and ascorbic acid.

9. The method of one of claims 4 to 8, further comprising the step of adding a disintegrant or a superdisintegrant prior to compressing.

## Patentansprüche

1. Gebrauchsfertige orale Zusammensetzung, umfassend: eine amorphe feste Dispersion, die Docetaxel, einen Hypromelloseacetatsuccinat-Träger (HPMCAS-Träger) und Natriumdodecylsulfat (SDS) umfasst, wobei das Docetaxel und das HPMCAS in der oralen Zusammensetzung in einem Verhältnis von 1 : 0,5 bis einschließlich 1 : 4 vorhanden sind; wobei das Docetaxel und das SDS in der oralen Zusammensetzung in einem Verhältnis von 1 : 5 bis 5 : 1 vorhanden sind, wobei die feste Dispersion eine sprühgetrocknete amorphe feste Dispersion oder ein durch Wirbelschichtgranulierung hergestelltes amorphes Granulat ist und wobei das Docetaxel in einer Konzentration von mindestens 6 Gew.-% der oralen Zusammensetzung vorhanden ist.

2. Zusammensetzung nach Anspruch 1, wobei die feste Dispersion mittels eines Feuchtgranulierungsverfahrens extragranulär mit intragranulären Hilfsstoffen gemischt wird, um eine Mischung zu bilden,
wobei die Mischung aus der Gruppe ausgewählt ist, die aus einem Schmiermittel, einem Füllstoff und einem Supersprengmittel besteht, um eine Tablettenmischung zu bilden, und wobei die Tablettenmischung zu Tabletten gepresst wird, um die gebrauchsfertige orale Zusammensetzung zu bilden.

3. Zusammensetzung nach Anspruch 1 oder 2, ferner umfassend ein Sprengmittel oder ein Supersprengmittel.

4. Verfahren zur Herstellung einer gebrauchsfertigen oralen Zusammensetzung, umfassend:
Formulieren einer Lösung, die Docetaxel, einen HPMCAS-Träger und SDS umfasst, wobei das Docetaxel und das HPMCAS in einem Verhältnis von 1 : 0,5 bis einschließlich 1 : 4 vorliegen; und wobei das Docetaxel und das SDS in einem Verhältnis von 1 : 5 bis einschließlich 5 : 1 vorliegen;
Auftragen der Lösung auf einen intragranulären Hilfsstoff unter Verwendung eines Wirbelschichtgranulierungssystems zum Bilden amorpher Granulate, die Docetaxel und den HPMCAS-Träger umfassen;
Trocknen der Vielzahl amorpher Granulate; und
Pressen der Vielzahl amorpher Granulate zum Bilden einer Tablette, die eine Docetaxel-Konzentration von mindestens 6 Gew.-% aufweist.

5. Verfahren nach Anspruch 4, wobei der intragranuläre Hilfsstoff mikrokristalline Cellulose ist.

6. Verfahren nach Anspruch 4 oder Anspruch 5, ferner umfassend einen Schritt des Zugebens eines extragranulären Hilfsstoffs vor dem Trocknen,
wobei der extragranuläre Hilfsstoff aus der Gruppe ausgewählt ist, die aus vernetztem Polyvinylpyrrolidon, amorphem Siliziumdioxid, Natriumstearylfumarat und mikrokristalliner Cellulose besteht.

7. Verfahren nach einem der Ansprüche 4 bis 6, wobei die Lösung ferner ein Tensid umfasst.

8. Verfahren nach einem der Ansprüche 4 bis 7, umfassend den Schritt des Zugebens eines Stabilisators vor dem Pressen, wobei der Stabilisator aus der Gruppe ausgewählt ist, die aus Zitronensäure und Ascorbinsäure besteht.

9. Verfahren nach einem der Ansprüche 4 bis 8, ferner umfassend den Schritt des Zugebens eines Sprengmittels oder eines Supersprengmittels vor dem Pressen.

## Revendications

1. Composition orale prête à l'administration, comprenant : une dispersion solide amorphe comprenant du docétaxel, un support d'acétate succinate d'hypromellose (HPMCAS, pour hypromellose acetate succinate en Anglais) ; et du dodécyl sulfate de sodium (SDS pour sodium dodecyl sulfate en Anglais), dans laquelle le docétaxel et l'HPMCAS sont présents dans la composition orale dans un rapport de 1:0,5 à 1:4 inclus ; dans laquelle le docétaxel et le SDS sont présents dans la composition orale dans un rapport de 1:5 à 5:1, dans laquelle la dispersion solide est une dispersion solide amorphe séchée par pulvérisation ou un granule amorphe produit par granulation en lit fluidisé et dans laquelle le docétaxel est présent à une concentration d'au moins 6 % en poids de la composition orale.

2. Composition selon la revendication 1 , dans laquelle la dispersion solide est mélangée de manière extragranulaire avec des excipients intragranulaires via un procédé de granulation humide pour former un mélange,
dans laquelle le mélange est choisi dans le groupe constitué d'un lubrifiant, d'une charge et d'un superdésintégrant pour former un mélange de comprimés, et dans laquelle le mélange pour comprimés est comprimé en comprimés pour former la composition orale prête à l'administration.

3. Composition selon l'une des revendications 1 ou 2, comprenant en outre un désintégrant ou un superdésintégrant.

4. Procédé de production d'une composition orale prête à l'administration, comprenant :
formuler une solution comprenant du docétaxel, un support de HPMCAS et du SDS, dans lequel le docétaxel et le HPMCAS sont dans un rapport de 1:0,5 à 1:4 inclus ; et dans lequel le docétaxel et le SDS sont dans un rapport de 1:5 à 5:1 inclus ;
appliquer la solution à un excipient intragranulaire à l'aide d'un système de granulation à lit fluidisé pour former des granules amorphes comprenant du docétaxel et le support de HPMCAS :
sécher la pluralité de granulés amorphes ; et
comprimer la pluralité de granules amorphes pour former un comprimé ayant une concentration en docétaxel d'au moins 6 % en poids.

5. Procédé selon la revendication 4, dans lequel l'excipient intragranulaire est la cellulose microcristalline.

6. Procédé selon la revendication 4 ou la revendication 5, comprenant en outre une étape d'ajout d'un excipient extragranulaire avant le séchage
dans lequel l'excipient extragranulaire est choisi dans le groupe constitué par la polyvinylpyrrolidone réticulée, le dioxyde de silicium amorphe, le stéaryl fumarate de sodium et la cellulose microcristalline.

7. Procédé selon l'une des revendications 4 à 6, dans lequel la solution comprend en outre un tensioactif.

8. Procédé selon l'une des revendications 4 à 7, comprenant l'étape consistant à ajouter un stabilisant avant la compression, dans lequel le stabilisant est choisi dans le groupe constitué de l'acide citrique et de l'acide ascorbique.

9. Procédé selon l'une des revendications 4 à 8, comprenant en outre l'étape consistant à ajouter un désintégrant ou un superdésintégrant avant la compression.
